# FASCICULE DE BREVET EUROPEEN

(11) **EP 0 686 396 B1**
(45) Date de publication et mention de la délivrance du brevet: **27.09.2000**
(21) Numéro de dépôt: 95100891.1
(22) Date de dépôt: 24.01.1995
(51) Int. Cl.: A61K 38/17, A61K 9/00, A23C 9/142, C07K 14/47

(54) **Composition entérale comprenant de la caséine micellaire native**
Micellares Kasein enthaltende enterale Zusammensetzung
Enteral composition containing native micellar casein

(30) Priorité: 25.02.1994 EP 94102851
(43) Date de publication de la demande: 13.12.1995
(73) Titulaire: SOCIETE DES PRODUITS NESTLE S.A., 1800 Vevey (CH)
(72) Inventeur: Jost, Rolf, CH-1814 La Tour-de-Peilz (CH)
(74) Mandataire: Archambault, Jean

(56) Documents cités:
- EP-A- 0 189 160
- DE-A- 3 801 391
- FR-A- 2 544 613
- US-A- 4 462 932
- DATABASE WPI Week 8827 Derwent Publications Ltd., London, GB; AN 88-189121 & SU-A-1 358 890 (ISTRINSKO DAIRY IND) , 15 Décembre 1987
- AUSTRALIAN JOURNAL OF DAIRY TECHNOLOGY, vol. 46, no. 2, Novembre 1991 pages 91-95, J.L. MAUBOIS 'New Applications of Membrane Technology in the Dairy Industry'
- JOURNAL OF DAIRY SCIENCE, vol. 74, no. 1, Janvier 1991 pages 50-57, S. SRILAORKUL ET AL 'Effect of Ultrafiltration of Skim Milk on Casein Micelle Size Distribution in Retentate'

## Description

La présente invention a trait à une composition entérale comprenant essentiellement de la caséine micellaire native comme source de protéine.

Une composition entérale est une composition liquide stérilisée destinée à l'alimentation de patients incapables de s'alimenter normalement. Elle est administrée par un tube nasal, buccal ou accédant directement à l'appareil digestif et doit apporter les trois éléments nutritifs de base, protéines, glucides et lipides dans un rapport adéquat et à une densité calorique élevée.

Le choix en matière de protéine pour ces compositions entérales est relativement limité. La plupart des protéines sont sensibles à la chaleur et coagulent lors de la stérilisation. Les caséinates font exception, et sont donc largement utilisés dans ce type de produit, partois associés à des protéines de soja. L'utilisation de poudre de lait écrémé comme source de caséine ne constitue pas une alternative aux caséinates car celle-ci contient trop de lactose, ce qui conduirait à une composition entérale de caractère laxatif et hyperosmotique.

Deux types de caséinates sont couramment utlisés: le caséinate de sodium ou de potassium (Na/K-caséinate) et le caséinate de calcium (Ca-caséinate).

Le Na/K-caséinate est soluble et présente une forte viscosité lorsque la concentration excède 5%. Cette forte viscosité est un inconvénient majeur pour la nutrition entérale, particulièrement nasale, qui utilise des tubages très fins. Les compositions entérales requièrent des concentrations élevées en protéine (de 5 à 10% en poids), afin que l'apport calorique se fasse en maintenant des proportions protéines/glucides/lipides équilibrées. La viscosité du Na/K-caséinate limite son usage aux compositions contenant moins de 6-7% de protéine.

Le Ca-caséinate n'est pas soluble, mais forme dans l'eau une dispersion colloïdale de viscosité relativement faible. Un inconvenient majeur de ce Ca-caséinate réside dans le fait qu'il provoque l'apparition de faux-goûts marqués dans les produits stérilisés.

La présente invention a pour objet de pallier aux inconvénients ci-dessus en proposant des compositions entérales comprenant essentiellement de la caséine micellaire native comme source de protéine.

La source de protéine est obtenue à partir de lait animal tel que le lait de vache, de chèvre ou de brebis. On la prépare par microfiltration de ce lait.

Cette protéine micellaire est obtenue par microfiltration de lait, le cas échéant suivie d'une diafiltration, sur une membrane présentant une porosité de 0,1 à 0,2 micromètre par exemple.

La microfiltration concentre sélectivement la caséine sans retenir les protéines sériques, ce qui présente l'avantage d'une bonne stabilité des protéines à la stérilisation.

De part ses propriétés remarquables, la caséine micellaire native ainsi préparée s'est avérée une source de protéine particulièrement adaptée aux compositions entérales. Elle ne présente aucun inconvénient des protéines précitées:
-ses qualités nutritionnelles sont équivalentes ou supérieures à celles des protéines traditionellement ultilisées,
-elle forme dans l'eau une dispersion colloïdale de faible viscosité permettant son utilisation à des concentrations élevées (5 à 10%) sans poser de problème d'écoulement dans les tubes les plus fins,
-elle possède une grande stabilité thermique et supporte donc parfaitement la stérilisation,
-à concentration égale, elle donne des solutions d'osmolarité plus faible que les caséinates,
-elle est totalement dépourvue de goût et
-elle donne des solutions d'une blancheur supérieure à celle des caséinates.

Les exemples ci-après illustrent l'invention. Les pourcentages et parties sont donnés en poids (g pour 100g de composition liquide), sauf indication différente.

### Exemple 1

### 1.1. Préparation de la caséine micellaire

On traite 300 kg de lait maigre de vache, pasteurisé à 72°C/15s, sur une installation de microfiltration (Techsep (R) module 1S 151) équipée de 3,4 m 2 de membrane M 14 (diamètre moyen des pores d'environ 0,15 micromètre) à une température allant de 50 à 55°C, jusqu'à ce l'on élimine 200 kg de perméat (ce qui correspond à une concentration d'un facteur volumique de 3). On diafiltre ensuite le rétentat avec de l'eau déminéralisée par élimination de 500 kg de perméat (correspondant à un facteur volumique de diafiltration de 5). Cette diafiltration a pour but de produire la caséine micellaire à une pureté protéique d'environ 85% avec moins de 1% de lactose résiduel sur matière sèche totale. On concentre alors le rétentat à un volume final de 50-70 l, ce qui garantit une teneur en matières sèches de 18-20%.
Le rétentat contient 185 g/kg de matières solides totales avec un taux de protéines de 84% sur matières sèches totales. Sa composition est la suivante en g/kg de liquide:

| | |
|---|---|
| Protéines | 164 |
| Lactose | 0,4 |
| Cendres | 15,6 |
| Matière grasse | 1,3 |
| Calcium | 6 |
| Potassium | 0,1 |
| Sodium | 0,04 |
| Phosphore | 1,7 |

Le fractionnement des protéines selon le schéma de Rowland (Rowland 1938; J. Dairy Res. 9,42-46) indique que, 96% de la protéine totale étant précipitable à pH 4,6, il s'agit de caséine. L'analyse d'une dispersion diluée de rétentat dans le "Malvern Particle Sizer" montre que la caséine est présente sous forme de particules de diamètre moyen 300 nanomètres (nm) et que la distribution des particules est relativement homogène (une famille seulement allant d'environ 100 à 400 nm). Cette image correspond parfaitement à celle que l'on obtient pour le lait maigre, ce qui indique la présence des micelles caractéristiques du lait.

### 1.2. Préparation d'une composition destinée à la nutrition entérale

2, 13 kg du rétentat précédent, contenant 19,5% de matières solides totales et correpondant à 350 g de protéines sont transférés dans une cuve thermostatisée à 55°C munie de moyens d'agitation. On ajoute à cette dispersion les ingrédients suivants sous forme solide (en g):

| | |
|---|---|
| Maltodextrine MD-02 | 300 |
| Saccharose | 150 |
| Tri-K-citrate H2O | 20,33 |
| MgCl2 6 H2O | 9,21 |
| NaCl | 5,84 |

Après dissolution des ingrédients dans le rétentat, de l'eau déminéralisée est ajoutée jusqu'à un poids total de 4825 g. A ce stade, le pH est contrôlé et, si nécessaire ajusté à la valeur de 6,8. On ajoute alors la phase grasse, soit 175 g d'huile de maïs contenant 2% (calculé sur l'huile) de lécithine de soja à titre d'émulgateur. Le mélange est homogénéisé à l'aide d'un homogénéisateur à deux étages, à 20 MPa au premier étage, puis à 5 MPa au second. L'émulsion est ensuite stérilisée par traitement UHT indirect (échangeur de chaleur) à 130°C/20 s, ou direct (injection directe de vapeur) à 148°C/5 s ou encore par stérilisation en boîte ou en poche à 120°C/6-8 min.

De la manière précédente, on prépare ainsi trois compositions entérales de densité énergétique identique, soit 4,184 kJ/ml (1 kcal/ml):

| Ingrédient (%) Protéines (%, p/v) | Composition A Caséine micellaire native 7 | Composition B Ca-caséinate 7 | Composition C Na/K-caséinate 7 |
|---|---|---|---|
| Lipides (huile de maïs) | 3,5 | 3,5 | 3,5 |
| GLucides (maltodextrines/saccharose) | 9,5 | 9,5 | 9,5 |
| Minéraux Ca | 0,21 | 0,2 | 0,07 |
| K | 0,16 | 0,15 | 0,18 |
| Na | 0,05 | 0,05 | 0,06 |
| Mg | 0,03 | 0,03 | 0,02 |
| P | 0,12 | 0,12 | 0,07 |
| Cl | 0,1 | 0,1 | 0,03 |

On compare les produits obtenus après stérilisation en boîte à 120°C/6 min du point de vue de leur teneur en protéines totales, viscosité dynamique (vitesse de cisaillement 1600 ^{s-1},température ambiante), osmolarité et propriétés organoleptiques:

| Composition | Protéines (total), % | Osmolarité mOsm/kgH2O | Viscosité mPa.s | Présence de faux-goûts |
|---|---|---|---|---|
| A | 7,56 | 392 | 6 | Non |
| B | 6,81 | 386 | 9 | Oui |
| C | 7,38 | 481 | 30 | Oui |

La composition A (caséine micellaire native) se distingue nettement de la composition C (Na/K-caséinate) par sa viscosité réduite et son osmolarité faible. Elle se distingue de la composition B (Ca-caséinate) sur le plan de la viscosité et du goût.

### Exemple 2

On prépare une composition entérale ayant une densité énergétique de 6,276 kJ/ml (1,5 kcal/ml) et 8% (p/v) de protéines à partir de poudre de lait écrémé "basse température", c'est à dire séché dans des conditions thermiques ménagées.

20 kg de poudre de lait écrémé basse température sont dispersés dans 100 kg d'eau déminéralisée à une température de 50-55°C. Cette dispersion correspondant à du lait maigre concentré deux fois est microfiltrée sur la même installation que dans l'exemple 1 en mode de diafiltration en passant de l'eau déminéralisée jusqu'à l'élimination de 600 kg de perméat. Le rétentat est ensuite encore concentré à environ 60 kg, ce qui représente une teneur en matières sèches de 21% avec un taux de protéines sur matières sèches de 82%.

Pour préparer la composition entérale, on mélange 2,323 kg de rétentat liquide, représentant 400 g de protéines totales à 55°C avec les ingrédients suivants (g):

| | |
|---|---|
| Maltodextrine MD-02 | 600 |
| Saccharose | 200 |
| Tri-K-citrate H2O | 20,33 |
| MgCl2 6 H2O | 9,21 |
| NaCl | 5,84 |

Après dissolution des ingrédients dans le rétentat, de l'eau déminéralisée est ajoutée jusqu'à un poids total de la dispersion de 4,7 kg. Le pH est alors ajusté à 6,8, puis 300g de phase grasse sont introduits, le poids total de la dispersion étant 5 kg. Après homogénéisation, puis stérilisation comme dans l'exemple 1, le produit présente une viscosité dynamique de 112 mPa.s (vitesse de cisaillement 1600 ^{s-1}, température ambiante). Il est d'apparence blanche, d'un goût sucré agréable et exempt de faux-goût.

## Revendications

1. Composition entérale comprenant essentiellement de la caséine micellaire native comme source de protéine.

2. Composition selon la revendication 1, dans laquelle la caséine micellaire native provient d'un lait animal, notamment d'un lait de vache, de chère ou de brebis.

3. Composition selon la revendication 1, dans laquelle la caséine micellaire native est obtenue par microfiltration, le cas échéant suivie de diafiltration, d'un lait animal.

4. Composition selon la revendication 1, dans laquelle la caséine micellaire native est présente à une concentration de 5-10% en poids.

5. Utilisation de caséine micellaire native comme source essentielle de protéine dans la fabrication d'une composition entérale.

6. Procédé de préparation d'une composition destinée à la nutrition entérale, caractérisé en ce que de la caséine micellaire est choisie comme source essentielle de protéine, ledit procédé consistant en ce que l'on microfiltre du lait écrémé, que, le cas échéant, l'on diafiltre ensuite de manière à isoler un rétentat, que l'on disperse dans le rétentat des glucides et des minéraux, que l'on ajoute à la dispersion une phase grasse et que l'on homogénéise, puis stérilise le mélange.

## Claims

1. Enteral composition substantially containing native micellar casein as a source of protein.

2. Composition according to claim 1, wherein the native micellar casein is derived from an animal milk, in particular cow's, goat's or sheep's milk.

3. Composition according to claim 1, wherein the native micellar casein is obtained by microfiltration, optionally followed by diafiltration, of an animal milk.

4. Composition according to claim 1, wherein the native micellar casein is present in a concentration of 5-10 % by weight.

5. Use of a native micellar casein as an essential source of protein in the production of an enteral composition.

6. Process for preparing a composition intended for enteral nutrition, characterized in that the micellar casein is chosen as an essential source of protein, the said process consisting of the microfiltration of skimmed milk which, optionally, is then subjected to diafiltration so as to isolate a retentate, in that carbohydrates and minerals are dispersed in the retentate, in that a fatty phase is added to the retentate and in that the mixture is homogenized and then sterilized.

## Patentansprüche

1. Enterale Zusammensetzung, die im wesentlichen micellares natives Casein als Proteinquelle enthält.

2. Zusammensetzung nach Anspruch 1, bei der das micellare native Casein aus einer Tiermilch stammt, insbesondere aus Kuhmilch, Ziegenmilch oder Schafmilch.

3. Zusammensetzung nach Anspruch 1, bei der das micellare native Casein durch Mikrofiltration, gegebenenfalls gefolgt von einer Diafiltration, einer Tiermilch, erhalten wurde.

4. Zusammensetzung nach Anspruch 1, bei der das micellare native Casein in einer Konzentration von 5-10 Gew.-% vorliegt.

5. Verwendung des micellaren nativen Caseins als wesentliche Proteinquelle bei der Herstellung einer enteralen Zusammensetzung.

6. Verfahren zur Herstellung einer Zusammensetzung, die für die enterale Ernährung bestimmt ist, dadurch gekennzeichnet, daß micellares Casein als wesentliche Proteinquelle gewählt wird, wobei das Verfahren darin besteht, daß man Magermilch mikrofiltriert, man gegebenenfalls anschließend so diafiltriert, daß man ein Retentat gewinnt, daß man in dem Retentat Kohlenhydrate und Mineralien dispergiert, daß man der Dispersion eine Fettphase zugibt und daß man homogenisiert, wonach man die Mischung sterilisiert.
